# EUROPEAN PATENT APPLICATION

(11) **EP 4 306 037 A1**
(43) Date of publication of application: **17.01.2024**
(21) Application number: 22767254.0
(22) Date of filing: 10.03.2022
(51) Int. Cl.: A61B 3/107

(54) **SIMPLE DIAGNOSIS ASSISTANCE DEVICE FOR KERATOCONUS AND ASTIGMATISM AND SO FORTH, AND DIAGNOSIS ASSISTANCE SYSTEM**

(30) Priority: 10.03.2021 JP 2021038152
(71) Applicant: Tsubota Laboratory, Inc., Tokyo 160-0016 (JP)
(72) Inventor: TSUBOTA Kazuo, Tokyo 160-0016 (JP); KOBASHI Hidenaga, Tokyo 160-0016 (JP); KATO Naoko, Tokyo 160-0016 (JP)
(74) Representative: Mewburn Ellis LLP
(86) International application number: PCT/JP2022/010745
(87) International publication number: WO 2022/191300

(57) **Abstract**

To provide a simple diagnosis assisting device that captures projected light projected onto an eyeball by using a mobile terminal and ring light in combination, and simply assists in diagnosis of keratoconus, astigmatism, and the like on the basis of obtained photographic data, and a diagnosis assisting system.

The above-described problem is solved by a simple diagnosis assisting device (10) for keratoconus and/or astigmatism, comprising a mobile terminal (10A) including a camera (2) and a transceiver function (3), and integrally or separately provided with a light source (1) for simultaneously projecting ring light onto both eyes. The light source (1) emits the ring light upon being attached to the mobile terminal (10A) in a case of being separately provided to the mobile terminal (10A), and emits the ring light from the mobile terminal (10A) in a case of being integrally provided to the mobile terminal (10A). The camera (2) simultaneously captures a projected image of both eyes onto which the ring light is projected. The ring light may be multiplex ring light or simplex ring light, and is configured in a ring shape by a plurality of point light sources or is configured in a ring shape by a single linear light source.

## Description

### Field of the Invention

The present invention relates to a simple diagnosis assisting device for keratoconus, astigmatism, and the like, and a diagnosis assisting system. More particularly, the present invention relates to a simple diagnosis assisting device that captures projected light projected onto an eyeball by using a mobile terminal and ring light in combination, and simply assists in diagnosis of keratoconus, astigmatism, and the like on the basis of obtained photographic data, and a diagnosis assisting system that uses diagnosis assistance data obtained by such a device to enable remote diagnosis assistance.

### BACKGROUND ART

There are various ophthalmic devices for eye diagnosis in correspondence with the purpose of diagnosis. For example, ophthalmic devices equipped with an optical system for measuring an axial length and an optical system for measuring a refractive power of an examined eye, ophthalmic devices equipped with an optical system for measuring an axial length and an optical system for measuring a corneal topography, and the like are known. Such ophthalmic devices are often large, and proposals have been made to reduce a size of an ophthalmic device, such as in Patent Document 1, for example.

As a device for analyzing a corneal topography, there is known a Placido-based corneal topography measuring device (Non-Patent Document 1). Measurement with this Placido-based corneal topography measuring device is made by analyzing a shape and a size, on the cornea, of a reflected image of light projected onto the eye. Then, the measurement principle is based on the fact that a size of a reflected image on a spherical surface is proportional to a radius of curvature of the spherical surface as long as a distance to the spherical surface onto which the light is projected is constant. Usually, the radius of curvature is calculated from the size of the reflected image of a Meyer ring projected onto the eye, and the refractive power of the cornea is determined by the radius of curvature and a refractive index of the cornea and utilized for diagnosis.

Patent Document 2 proposes a technique that utilizes advanced mobile processing device technologies (camera, light source, extension/editing, software application) and aims to link the technologies to improvements in patient eye care worldwide. This technique provides a mobile processing device and a system that uses the mobile processing device, and the mobile processing device includes a camera lens, a light source, and a processor configured to process captured and received images.

### PRIOR ART DOCUMENTS

### Non-Patent Documents

Non-Patent Document 1: Shizuka Koh, "Principles and future prospects of Placido-based corneal topography," Japanese Journal of Visual Science, Vol. 37, No. 4 (December 2016).

### Patent Documents

Patent Document 1: Japanese Laid-Open Patent Application Publication No. 2016-77774Patent Document 2: US Patent No. 10129450

### SUMMARY OF THE INVENTION

### Problems to be Solved by the Invention

A Placido-based corneal topography measuring device, similar to other ophthalmic devices, is an installation-type device installed in a medical institution as an ophthalmic device having a certain size. Further size reduction is expected for such corneal topography measuring devices as well. The corneal topography measuring device is a necessary device in terms of performing a diagnosis accurately, and thus there is little demand for reducing the size of the device to the extent of being transportable to any location.

Further, the mobile processing device of Patent Document 2 is equipped with a Meyer-ring generating light source in a mobile terminal, and has a structural form that can be easily carried by a physician and easily applied to capture images of the eyes of a patient and process the captured images. However, the patient must be imaged and examined by the physician and the images are captured one eye at a time, making the device unusable in private homes, remote areas, remote islands, developing countries, and the like in a case in which a physician is not available.

On the other hand, a patient with an eye disorder or the like wants continuous follow-up after being diagnosed by a physician at a hospital, and a physician wants to identify deterioration of the disorder or the like through continuous follow-up of the symptoms of the patient. If, particularly in private homes, remote areas, remote islands, developing countries, and the like where equipment is not available, supplementary data that serves as a reference for eye diagnosis could be acquired and transmitted to a physician at a remote location by some simple device, the current progress could be checked by the physician at a distant location. Further, such a simple device would also allow the physician to determine whether to have the patient visit the physician's office at the distant location for an initial diagnosis or a detailed examination.

The present invention has been made to solve the above-described problems, and an object thereof is to provide a simple diagnosis assisting device that captures projected light projected onto an eyeball by using a mobile terminal and ring light in combination, and simply assists in diagnosis of keratoconus, astigmatism, strabismus, and the like on the basis of obtained photographic data, and a diagnosis assisting system that uses diagnosis assistance data obtained by such a device to enable remote diagnosis assistance.

### Means for Solving the Problems

(1) A simple diagnosis assisting device according to the present invention is a simple diagnosis assisting device for keratoconus and/or astigmatism, comprising a mobile terminal including a camera and a transceiver function, and integrally or separately provided with a light source for simultaneously projecting ring light onto both eyes. The light source emits the ring light upon being attached to the mobile terminal in a case of being separately provided to the mobile terminal, and emits the ring light from the mobile terminal in a case of being integrally provided to the mobile terminal. The camera simultaneously captures a projected image of both eyes onto which the ring light is projected.

According to this invention, the mobile terminal is integrally or separately provided with the light source, making it possible to simultaneously and readily capture the ring light projected onto both eyes by the camera and, on the basis of obtained photographic data, quickly and simply perform diagnosis of keratoconus, astigmatism, strabismus, and the like of each eye. The mobile terminal includes at least the camera and the transceiver function and is integrally or separately provided with the light source, and thus is a particularly effective tool capable of simply acquiring photographic data in private homes, remote areas, remote islands, developing countries, and the like where equipment is not available, making it possible to have a physician at a distant location look at the photographic data and thus receive a simple diagnosis by the physician, and have the physician look at current conditions during a follow-up examination or the like. Further, the camera simultaneously captures a projected image of both eyes onto which the ring light is projected, making it possible to simultaneously capture an image of both eyes, in contrast to the capturing of images one eye at a time with a device in the related art. This way, with imaging completed once, the image can be conveniently captured even in private homes, remote areas, remote islands, developing countries, and the like in a case in which equipment is not available or in a case in which another person is not available to capture the images.

In the simple diagnosis assisting device according to the present invention, the light source is disposed toward a side permitting visual confirmation of a display of the mobile terminal.

According to this invention, the device has a structural form in which the light source is disposed toward the side that permits visual confirmation of the display of the mobile terminal, making it possible to capture an image of oneself (take a selfie) after confirming on one's own that the ring light is reflected in both eyes by looking at the display of the mobile terminal.

In the simple diagnosis assisting device according to the present invention, the ring light is multiplex ring light or simplex ring light.

According to this invention, the ring light may be multiplex ring light or may be simplex ring light and thus, by capturing the projected light of such ring light projected onto both eyes and performing image processing on the photographic data, it is possible to analyze the topography of each eye and simply utilize the data as a reference for diagnosing keratoconus, astigmatism, strabismus, and the like.

In the simple diagnosis assisting device according to the present invention, the ring light is configured in a ring shape by a plurality of point light sources or is configured in a ring shape by a single linear light source.

According to this invention, the ring light may be configured in a ring shape by a plurality of point light sources or may be configured in a ring shape by a single linear light source, making it possible to analyze the topography of each eye simply by performing processing corresponding to the form of the ring light, and simply utilize the data as a reference for diagnosing keratoconus, astigmatism, strabismus, and the like. It should be noted that such processing may be performed by application software built into the mobile terminal, or may be performed by application software of a computer terminal that receives the photographic data transmitted from the mobile terminal.

In the simple diagnosis assisting device according to the present invention, the ring light has a color at a predetermined position on a contour thereof that is different from the color at other positions.

According to this invention, the ring light includes a specific color at a predetermined position on a contour thereof that is different from the color at other positions and, in a case in which the contour is given clock coordinates, for example, positions such as 0:00, 3:00, 6:00, and 9:00 are imparted with a specific color, making it possible to specify coordinate positions and effectively utilize the positions in photographic data analysis. In particular, in the simple diagnosis assisting device of the present invention with which an image is taken by a selfie, such coordinate positions may be an aid in imaging and are thus preferred.

In the simple diagnosis assisting device according to the present invention, diagnosis assistance for the keratoconus, the astigmatism, and other ocular conditions is provided from a topography of the eyeball obtained on the basis of photographic data captured by the camera.

According to this invention, it is possible to easily provide diagnosis assistance for keratoconus, astigmatism, and other ocular conditions. Specifically, the captured photograph is analyzed as photographic data, and a deformed topography and other analytical information obtained by processing the photographic data are utilized in assisting in the diagnosis of keratoconus, astigmatism, and other ocular conditions.

In the simple diagnosis assisting device according to the present invention, the mobile terminal incorporates an application for adjusting a contrast and/or an illuminance of the ring light in a case in which the light source is integrally provided to the mobile terminal, and the light source incorporates a device for adjusting the contrast and/or the illuminance of the ring light in a case in which the light source is separately provided to the mobile terminal.

According to this invention, in a case in which the light source is integrally provided to the mobile terminal, it is possible to adjust the contrast and/or the illuminance of the ring light by an internal application and capture the projected light thus adjusted. Further, in a case in which the light source is separately provided to the mobile terminal, it is possible to adjust the contrast and/or the illuminance of the ring light by an adjusting device built into the light source separately provided. In both of these cases, the captured image is easier to view, making diagnosis assistance based on the transmitted data easier.

(2) A diagnosis assisting system according to the present invention comprises the above-described simple diagnosis assisting device according to the present invention, and a terminal that receives diagnosis assistance information transmitted from the simple diagnosis assisting device.

According to this invention, the simple diagnosis assisting device can form photographic data that can be simply utilized in the diagnosis and the like of keratoconus, astigmatism, strabismus, and the like and thus, by transmitting the photographic data, the transmitted data can be received at a distant location. As a result, the diagnosis assisting system is particularly effective in private homes, remote areas, remote islands, developing countries, and the like in a case in which equipment is not available or in a case in which another person is not available to capture the image, making it possible to have a physician at a distant location look at the measurement data and thus receive the physician's findings, and have the physician look at current conditions during a follow-up examination or the like.

### Effect of the Invention

According to the present invention, it is possible to provide a simple diagnosis assisting device that captures projected light projected simultaneously onto both eyes by using a mobile terminal and ring light in combination, and simply assists in diagnosis of keratoconus, astigmatism, strabismus, and the like on the basis of obtained photographic data, and a diagnosis assisting system that uses diagnosis assistance data obtained by such a device to enable diagnosis assistance in private homes, remote areas, remote islands, developing countries, and the like in a case in which equipment is not available or in a case in which another person is not available to capture the images.

### BRIEF DESCRIPTION OF THE DRAWINGS

Figs. 1A and 1B are schematic views illustrating an example of a simple diagnosis assisting device according to the present invention, Fig. 1A illustrating an example of a light source illustrating a ring shape and separately attached toward one's own side, and Fig. 1B being an example of the light source having a ring shape and separately attached toward the other side.
Figs. 2A and 2B illustrate an example of a structural form of an attaching part for attaching a light source separately provided to a mobile terminal, Fig. 2A being a perspective view of a display screen side, and Fig. 2B being a perspective view of a side opposite to the display screen.
Fig. 3 illustrates a schematic view illustrating another example of a simple diagnosis assisting device according to the present invention, and is an example of an integrated light source with ring light displayed on the display screen.
Figs. 4A and 4B illustrate examples of forms of the ring light, Fig. 4A illustrating an example of duplex ring light, and Fig. 4B illustrating an example of simplex ring light.
Figs. 5A and 5B illustrate examples in which, in multiplex ring light or simplex ring light, each ring light is configured in a ring shape by a plurality of point light sources, and each ring light is configured in a ring shape by a single linear light source, respectively.
Figs. 6A and 6B illustrate examples in which a shape of the ring light is a circular ring shape and a rectangular ring shape, respectively.
Fig. 7 is a perspective view illustrating another example of the light source including a duplex ring.
Fig. 8 illustrates an example of a case in which a projected image of both eyes onto which the ring light is projected is simultaneously captured.
Figs. 9A to 9D illustrate an example of a diagnosis mode that utilizes a diagnosis assisting system according to the present invention.
Fig. 10 illustrates an example of a wellness system that utilizes the diagnosis assisting system according to the present invention.
Figs. 11A to 11C show examples of the projection of rings on corneal surfaces of both eyes of a subject by a selfie by using the simple diagnosis assisting device according to the present invention.
Fig. 12 shows examples of processing of an obtained image.
Fig. 13 shows an example of analysis of an obtained ring image.
Fig. 14 shows examples of images captured by using rings with different quantities of LEDs.

### Embodiments of the Invention

A simple diagnosis assisting device and a diagnosis assisting system according to the present invention will now be described below with reference to the drawings. The present invention is not limited to the embodiments and examples below, and various modifications can be made as long as the gist of the present application is included.

### [Simple Diagnosis Assisting Device]

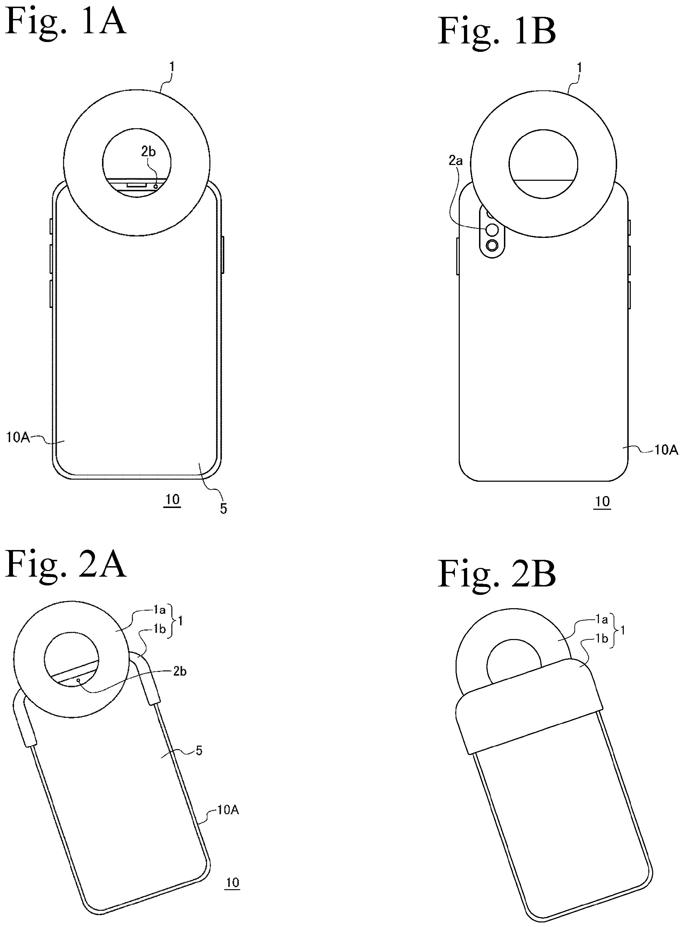

A simple diagnosis assisting device 10 according to the present invention is, as illustrated in Figs. 1A and 1B and Figs. 2A and 2B, a simple diagnosis assisting device 10 for keratoconus and/or astigmatism, including a mobile terminal 10A including a camera 2 and a transceiver function 3, and integrally or separately provided with a light source 1 for simultaneously projecting ring light onto both eyes. The light source 1 emits the ring light upon being attached to the mobile terminal 10A in a case of being separately provided to the mobile terminal 10A, and emits the ring light from the mobile terminal 10A in a case of being integrally provided to the mobile terminal 10A. The camera 2 simultaneously captures the projected images of both eyes onto which the ring light is projected.

This simple diagnosis assisting device 10, with the mobile terminal 10A integrally or separately provided with the light source 1, can simultaneously and readily capture the ring light projected onto both eyes with the camera 2 and, on the basis of obtained photographic data, quickly and simply perform diagnosis of keratoconus, astigmatism, strabismus, and the like of each eye. The mobile terminal 10A includes at least the camera 2 and the transceiver function 3 and is integrally or separately provided with a light source 1, and thus is a particularly effective tool capable of simply acquiring photographic data in private homes, remote areas, remote islands, developing countries, and the like where equipment is not available, making it possible to have a physician at a distant location look at the photographic data and thus receive a simple diagnosis by the physician, and have the physician look at current conditions during a follow-up examination or the like. Further, the camera 2 simultaneously captures a projected image of both eyes onto which the ring light is projected, making it possible to simultaneously capture an image of both eyes, in contrast to the capturing of images one eye at a time with a device in the related art. This way, with imaging completed once, the image can be conveniently captured even in private homes, remote areas, remote islands, developing countries, and the like in a case in which equipment is not available or in a case in which another person is not available to capture the images.

Hereinafter, each component will be described.

### [Mobile Terminal]

The mobile terminal 10A usually includes at least the camera 2 and the transceiver function 3, as illustrated in Figs. 1A and 1B and Figs. 2A and 2B. The transceiver function 3 is a function normally provided to the mobile terminal 10A that functions as a mobile communication terminal, but a transceiver function itself is provided even in a case in which no communication contract has been made and data cannot be transmitted or received. The camera 2 is also normally provided to the mobile terminal 10A and includes a main camera provided on a side opposite to a display screen (also referred to as "display") and a sub-camera provided on the side of the display screen. The main camera is used in a case of capturing an image while viewing a subject not on one's own side on the display screen, and the sub-camera is used in a case of capturing an image while viewing a subject on one's own side on the display screen. In the present invention, an image is captured (a selfie is taken) of both of one's own eyes, and thus the sub-camera is used. A light source is also normally provided to the mobile terminal 10A and is utilized as a flash when taking a photograph, for emergency lighting, and the like. However, the light source 1 applied in the present invention is not this light source. The light source 1 applied in the present invention is a light source integrally or separately provided to the mobile terminal 10A.

### (Light source)

The light source 1 is not a light source that uses, as is, the light source 1 (1a, 1b) provided to the mobile terminal 10A in advance, and is a light source integrally or separately provided. In a case in which the light source 1 is separately provided to the mobile terminal 10A, the ring light is emitted upon being attached to the mobile terminal 10A. On the other hand, in a case in which the light source 1 is integrally provided to the mobile terminal 10A, the ring light is emitted from a display screen 5 of the mobile terminal 10A.

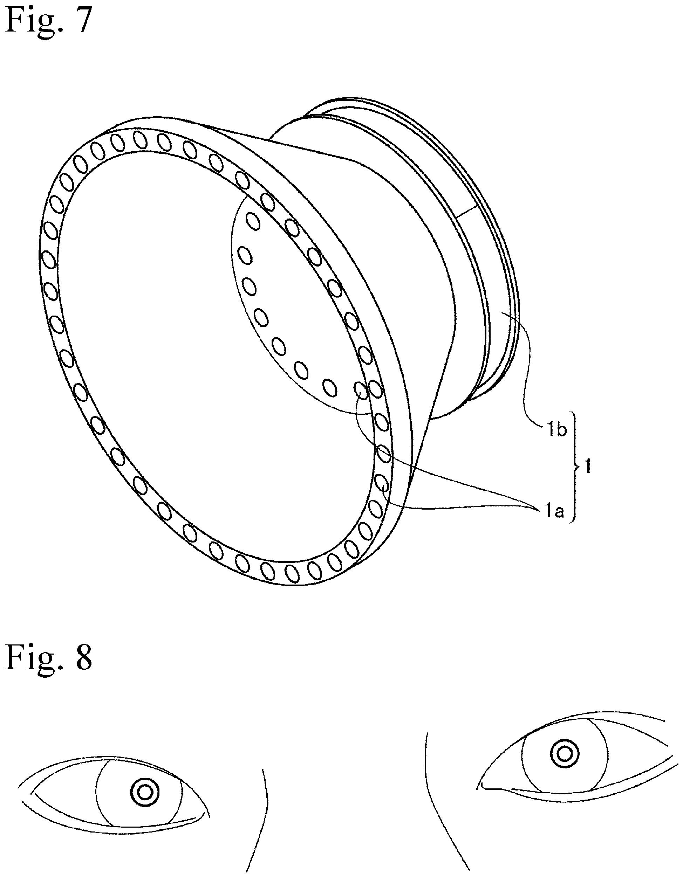

Representative examples of the light source 1 separately provided include, as illustrated in Figs. 1A and 1B and Fig. 7, the light source 1a attached as a separate member to an upper portion of the mobile terminal 10A. Fig. 1A illustrates an example of the light source 1a having a ring shape and separately attached toward one's own side, and Fig. 1B illustrates an example of the light source 1a having a ring shape and separately attached toward the other side. The light sources 1a in Fig. 1A and Fig. 1B differ in terms of whether an orientation of installation is toward one's own side or the other side, but are the light sources 1a having the same ring shape. Further, the light source 1 illustrated in Fig. 7 has a form such as cone or trumpet shape, with the light source 1a having a large-diameter ring shape disposed at an end portion and the light source 1a having a small-diameter ring shape disposed on an inner peripheral surface.

The light source 1 separately provided includes, as illustrated in Figs. 2A and 2B and Fig. 7, the attaching part 1b for attachment to the mobile terminal 10A. The attaching part 1b is a structural member for attaching the light source 1a to the upper portion of the mobile terminal 10A on the camera side and, while a structural form thereof is not particularly limited, can be exemplified by a structural form that allows insertion of the upper portion of the mobile terminal 10A without causing the camera or the display screen to be hidden, as illustrated in Figs. 2A and 2B. Although the orientation of installation of the light source 1a separately provided can be either toward one's own side or toward the other side, because it is desirable to be able to take a selfie in the present invention, the structural form of the attaching part 1b is preferably a form that facilitates attachment toward one's own side. Specifically, as illustrated in Fig. 1A and Fig. 7, the light source 1a is attached with the orientation of installation toward one's own side, and thus the attaching part 1b need not cover a sub-camera 2b for imaging, and may have a structural form that widely covers a flat surface side on the side without the display screen 5. It should be noted that, as illustrated in Fig. 1B, it is also possible to set the orientation of installation of the light source 1a toward the other side and, in this case, the attaching part 1b needs to have a structural form that does not cover a main camera 2a for imaging and does not excessively cover the display screen 5, and preferably has the structural form in which the attaching part 1b engages with a frame of the mobile terminal 10A.

It should be noted that the light source 1a separately provided is not limited to the light source having a ring shape, illustrated in Figs. 1A and 1B, Figs. 2A and 2B, and Fig. 7, and may have a form in which a liquid crystal panel 1c capable of emitting ring light is attached to the upper portion of the mobile terminal 10A. Further, the light source 1 is connected to the mobile terminal 10A by a wire, such as a USB, or by wireless communication, such as WiFi.

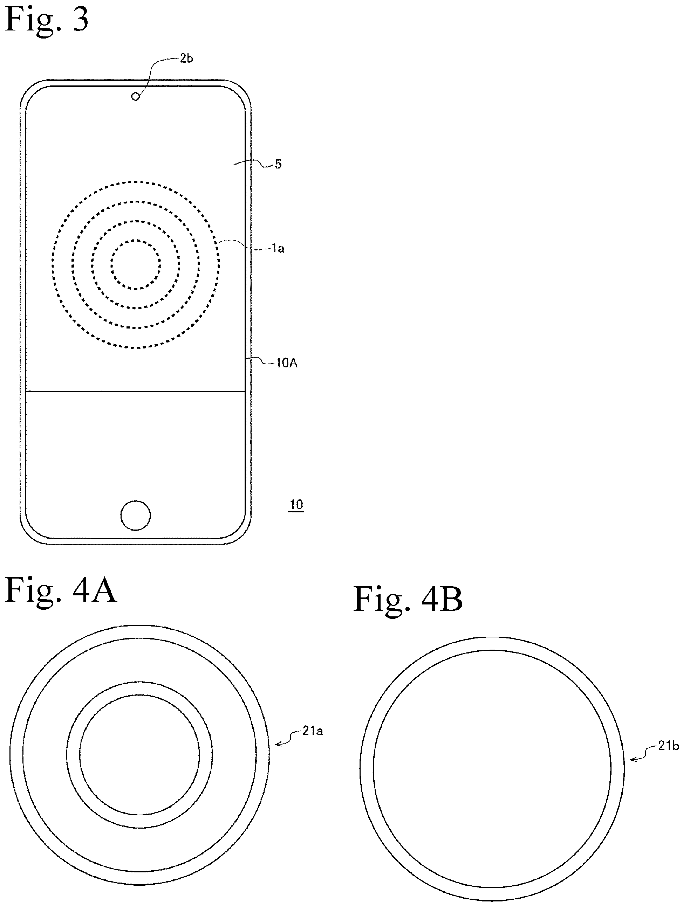

The light source 1a integrally provided emits the ring light from the display screen 5 of the mobile terminal 10A, as illustrated in Fig. 3. The ring light in this case is displayed on the display screen 5 by application software that forms the ring light. In a case in which the light source 1a integrally provided is used, the ring light displayed on the display screen is viewed on one's own, and a selfie is taken by using the sub-camera 2b, and thus, in the invention of the present application, the light source 1a integrally provided is preferably applied. It should be noted that, in a case of using the light source 1a integrally provided, the light source separately provided is not required and the attaching part 1b is also not required.

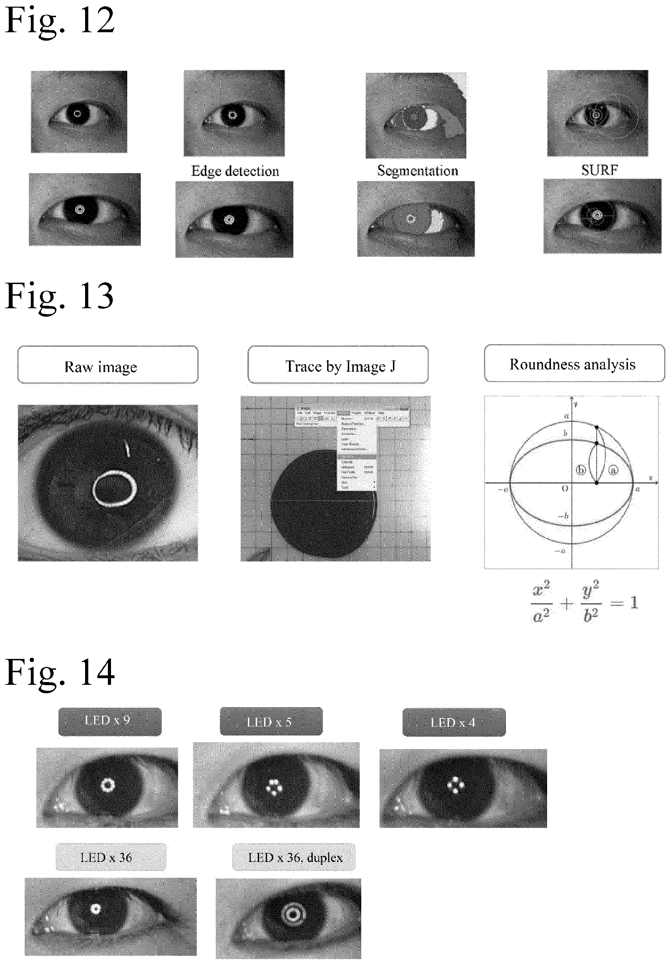

In a case in which the ring light is emitted from the display screen 5 of the mobile terminal 10A, images in which an emitting form is changed from a quadruplex ring illustrated in Fig. 3 to a duplex ring, a triplex ring, or a simplex ring may be obtained in combination, or images in which the emission form is changed to ring light obtained by changing a number of light-emitting diodes (LEDs) in Fig. 14 described below may be obtained in combination. With a plurality of images having different ring light forms thus obtained, these images can be processed and quantified, and thus utilized as various diagnostic materials.

### (Ring light)

The ring light is projected onto both eyes. The ring light may be multiplex ring light (which can be rephrased as "two or more ring lights" or "a plurality of ring lights"), or may be simplex ring light. The ring light projected onto both eyes may be multiplex or may be simplex, and this projected ring light is captured by the camera to generate photographic data and the photographic data is subjected to image processing, making it possible to analyze the topography of the eye and simply utilize the data as a reference for diagnosing keratoconus, astigmatism, strabismus, and the like. The diagnosis of keratoconus, astigmatism, strabismus, and the like can be made on the basis of evaluation results obtained by utilizing the measurement principle of a Placido-based corneal topography measuring device known in Non-Patent Document 1 and the like. In this measurement principle, a radius of curvature is calculated from a size of the ring light projected onto a surface of each eyeball, and a refractive power of the cornea is determined by the radius of curvature and a refractive index of the cornea and utilized for diagnosis. It should be noted that the image processing of the photographic data may be performed by application software built into the mobile terminal 10A, or may be performed by application software of a computer terminal that receives the photographic data transmitted from the mobile terminal 10A.

Figs. 4A and 4B illustrate examples of forms of the ring light. Fig. 4A illustrates an example of duplex ring light 21a, and Fig. 4B illustrates an example of simplex ring light 21b. The ring light need not be duplex such as in Fig. 4A, and may be triplex or greater.

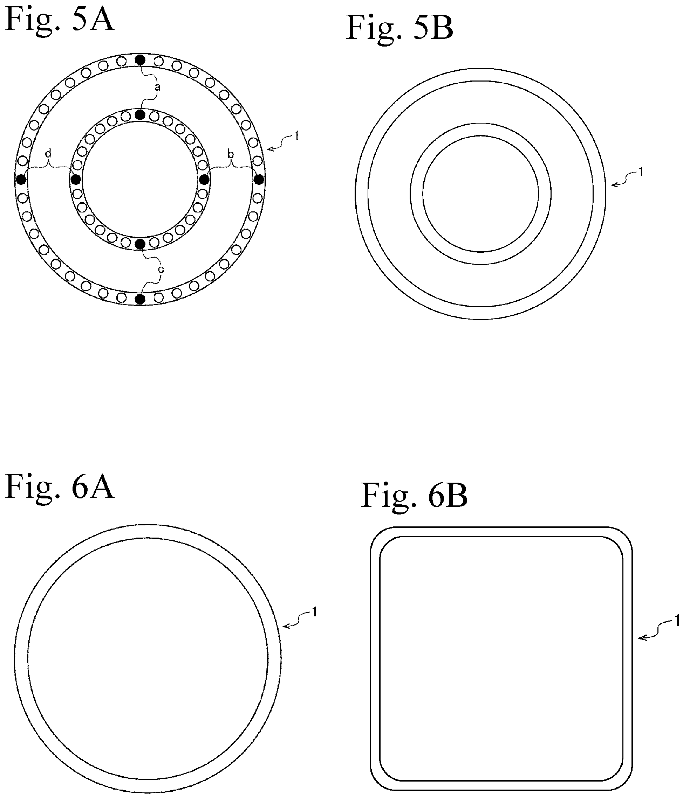

The multiplex ring light or simplex ring light may be configured in a ring shape by a plurality of point light sources or may be configured in a ring shape by a single linear light source. Fig. 5A illustrates an example in which each ring light is configured in a ring shape by a plurality of point light sources, and Fig. 5B illustrates an example in which each ring light is configured in a ring shape by a single linear light source. As the point light source, an LED is used, and the LEDs are disposed in a ring shape to constitute the ring light illustrated in Fig. 5A. As the linear light source, a fluorescent tube or LED tube is disposed in a ring shape to configure the ring light illustrated in Fig. 5B. It should be noted that Fig. 7 also illustrates an example in which each ring light is configured in a ring shape by a plurality of point light sources, but the configuration is not limited thereto.

The ring light, as illustrated in Figs. 5A and 5B, may have a color at predetermined positions (a, b, c, d) on a contour thereof that is different from the color at other positions. This way, in a case in which the contour is given clock coordinates, positions such as 0:00, 3:00, 6:00, and 9:00 (a, b, c, d) can be specified and effectively utilized in photographic data analysis. While the colors of the predetermined positions are not particularly limited, the colors can be realized by arranging LEDs having different colors at the positions (a, b, c, d). In particular, in the simple diagnosis assisting device 10 of the present invention with which an image is taken by a selfie, such coordinate positions may be an aid in imaging and are thus preferred.

A shape of the ring light may be a circular ring shape exemplified in Fig. 6A, or may be a rectangular ring shape exemplified in Fig. 6B. Examples of the circular ring shape include a perfect circle, an ellipse, and the like. In a case in which the shape of the light source is circular, a circular ring shape is preferred. Examples of the rectangular ring shape include an equilateral triangle, an equilateral quadrangle, an equilateral pentagon, and other polygons. In a case in which the shape of the light source is rectangular, a rectangular ring shape is preferred. Examples of the rectangular light source include a case in which an outer edge portion of the mobile terminal 10A such as a smartphone is the light source, and a case in which a peripheral edge portion of a protective cover or the like that protects the mobile terminal 10A is the light source. The photographic data obtained by projecting the ring light having such a shape is processed by application software and the topography of the eye is analyzed.

### (Camera)

The camera 2 is normally provided to the mobile terminal 10A and includes the main camera 2a provided on a side opposite to the display screen (also referred to as "display") and the sub-camera 2b provided on the display screen side. The main camera 2a is used in a case of capturing an image while viewing a subject not on one's own side on the display screen 5. However, because a selfie is the desired mode in the present invention, the sub-camera 2b, which allows a selfie to be taken while viewing a subject on one's own side on the display screen 5, is preferably used. It should be noted that the light source 1 is provided in a position that does not interfere with the imaging by the camera 2.

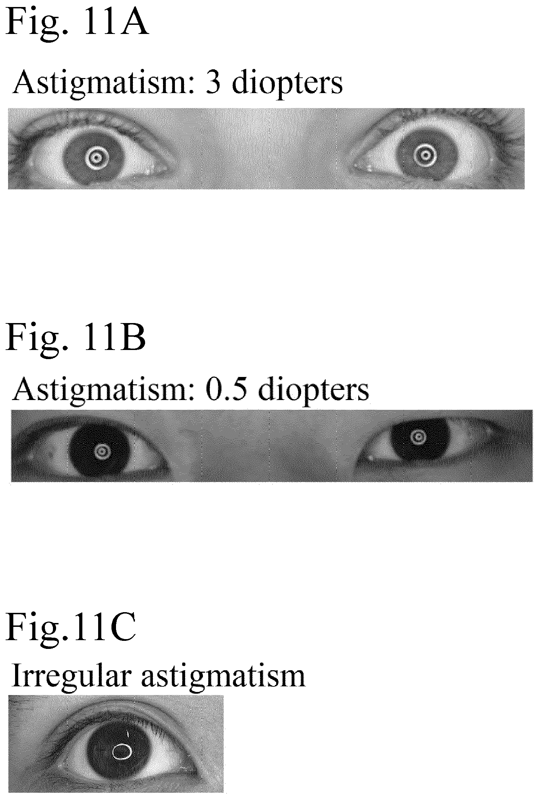

The camera 2, in the present invention, simultaneously captures a projected image of both eyes onto which the ring light is projected (refer to Fig. 8, Figs. 11A to 11C, and the like). In contrast to the capturing of images one eye at a time with a device in the related art, the camera 2 simultaneously captures a projected image of both eyes in the present invention, and thus imaging is completed once. As a result, the image can be conveniently captured even in private homes, remote areas, remote islands, developing countries, and the like in a case in which equipment is not available or in a case in which another person is not available to capture the image.

The topography of the eyeball can be analyzed by the image processing of the photographic data captured by the camera 2. As a result, it is possible to provide diagnosis assistance for keratoconus, astigmatism, and other ocular conditions. Specifically, the captured photograph is analyzed as photographic data, and a deformed topography and other analytical information obtained by processing the photographic data are utilized in assisting in the diagnosis of keratoconus, astigmatism, and other ocular conditions.

### (Transceiver function)

The transceiver function 3 is a function normally provided to the mobile terminal 10A that functions as a communication device. It should be noted that the mobile terminal 10A includes the transceiver function itself, even in a case in which no communication contract has been made and data cannot be transmitted or received. This transceiver function 3 makes it possible to transmit the captured photographic data to a remote computer terminal or the like and, in a case in which the photographic data is operated and processed by the mobile terminal 10A, transmit the operated data to a remote computer terminal or the like. The transceiver function may be a dedicated application for photographic data or may be a general-purpose application of the mobile terminal 10A, such as e-mail, and is not particularly limited.

### (Light source adjustment and the like)

As illustrated in Fig. 3, the mobile terminal 10A may incorporate an application for adjusting a contrast and/or an illuminance of the ring light in a case in which the light source 1 is integrally provided to the mobile terminal 10A. This way, the captured image is easier to view, making diagnosis assistance based on the transmitted data easier.

Further, the mobile terminal 10A may include an application for alignment adjustment during imaging, and an application for centering adjustment. This way, an image can be captured in an easy-to-diagnose state.

Further, as illustrated in Figs. 1A and 1B, the light source 1 may incorporate a device for adjusting (not illustrated) the contrast and/or the illuminance of the ring light in a case in which the light source 1 is separately provided to the mobile terminal 10A. This way, the captured image is easier to view, making diagnosis assistance based on the transmitted data easier. It should be noted that, in a case in which the light source 1 does not incorporate an adjusting device, the mobile terminal 10A may incorporate an adjusting application, and the adjusting application may control the contrast and the illuminance of the light source 1.

### [Diagnosis Assisting System]

A diagnosis assisting system according to the present invention includes the above-described simple diagnosis assisting device 10 according to the present invention, and a terminal that receives diagnosis assistance information transmitted from the simple diagnosis assisting device 10 (referred to as "diagnosis assisting terminal"). The simple diagnosis assisting device 10 can form photographic data that can be simply utilized in the diagnosis and the like of keratoconus, astigmatism, strabismus, and the like and thus, by this diagnosis assisting system, the photographic data actually obtained is transmitted from the mobile terminal 10A to the diagnosis assisting terminal, making it possible to receive the transmitted data at a distant location. As a result, the diagnosis assisting system is particularly effective in private homes, remote areas, remote islands, developing countries, and the like where equipment is not available, making it possible to have a physician at a distant location look at the measurement data and thus receive the physician's findings, and have the physician look at current conditions during a follow-up examination or the like. It should be noted that the simple diagnosis assisting device 10 has already been described in detail, and thus a description thereof will be omitted here.

The diagnosis assisting terminal is preferably a computer terminal or the like used by a physician at a medical institution for medical treatment, but may be a mobile terminal such as a smartphone or tablet terminal used by a physician.

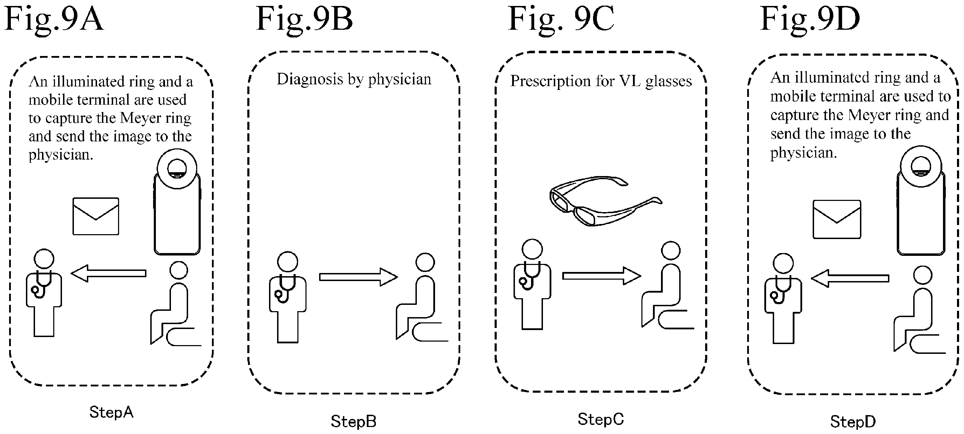

Figs. 9A to 9D illustrate an example of a diagnosis mode utilizing the diagnosis assisting system according to the present invention. In the diagnosis assisting system according to the present invention, first, in step A of Fig. 9A, a selfie is taken of both eyes with the simple diagnosis assisting device 10, and the processed data is transmitted to the medical institution as necessary. A simple diagnosis is performed by the physician on the basis of the received data. The simple diagnosis based on the transmitted data can be subjected to a mass examination, a re-examination, a follow-up examination, or the like, for example. By going through the simple diagnosis, the physician can determine whether or not a visit to the hospital for direct medical care is necessary, and continually determine remote guidance, follow-up examinations, and the like without hospital visits. As a result, the diagnosis assisting system can be easily used even in private homes, remote areas, remote islands, developing countries, and the like in a case in which equipment is not available or in a case in which another person is not available to capture the image, making it possible to have a physician at a distant location look at data and thus receive the physician's findings.

In step B in Fig. 9B, in a case of a visit to the hospital for direct medical care, the physician can perform a detailed examination with the ophthalmic device installed at the medical institution and determine the subsequent treatment plan and the like.

In step C in Fig. 9C, if the diagnosis reveals signs of keratoconus, for example, an effective procedure effective for treating such keratoconus or suppressing a progression thereof can be performed. For example, the eyeglasses-type violet-light irradiation device of Patent No. 6175210 already proposed by the present inventors or the like can be applied.

In step D in Fig. 9D, subsequently the physician can continually perform follow-up examinations and continually determine remote guidance, follow-up examinations, and the like without hospital visits.

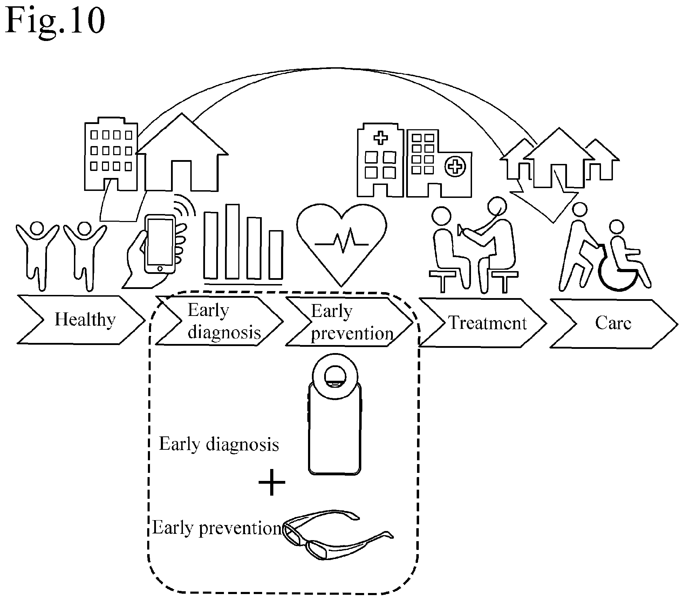

Fig. 10 illustrates an example of a wellness system that utilizes the diagnosis assisting system according to the present invention. The diagnosis assisting system enables early diagnosis and early prevention for people in distant locations such as private homes, remote areas, remote islands, and developing countries where equipment is not available, through simple diagnosis by a physician, making it possible to have a physician determine whether or not a visit to the hospital for direct medical care is necessary, and continually determine remote guidance, follow-up examinations, and the like without hospital visits.

### Examples

Hereinafter, the present invention will be more specifically described with reference to actually captured images.

Figs. 11A to 11C show examples of the projection of rings on corneal surfaces of both eyes of a subject by a selfie. Fig. 11A shows an example of a duplex ring projection, and shows a case in which the astigmatism of the subject is 3 diopters. Fig. 11B also shows an example of a duplex ring projection, and shows a case in which the astigmatism of the subject is 0.5 diopters. Fig. 11C shows an example of a simplex ring projection, and shows an example in which the subject has irregular astigmatism. While the rings in the images of subjects with high astigmatism or irregular astigmatism were ellipse-shaped, those with low astigmatism were perfect-circle-shaped, making it possible to simply determine the difference between the two. Such images, by being transmitted to a physician and subjected to image processing, can be diagnosed by the physician and quantified.

Fig. 12 shows examples of the processing of an obtained image, and shows an example in which a leftmost captured image is processed by edge detection, segmentation, and speeded-up robust features (SURF). From these, it is understood that the processing is different from that of clinical corneal topography, and a simple diagnosis can be more efficiently made by quantification through image processing.

Fig. 13 shows an example of analysis of an obtained ring image. This is an example in which a roundness analysis is performed. In the example in Fig. 13, the roundness analysis reveals an ellipse, and thus the determination can be made that the eye is regular astigmatic. The roundness analysis makes it possible to simply determine suspicion of keratoconus in a case in which ellipticity or irregularity is present.

Fig. 14 shows examples of images captured by using rings with different quantities of LEDs. By changing the number and the arrangement of the LEDs, it can be observed that the images of the ring light projected onto the eyeball each have characteristics. The images are processed and quantified in correspondence with the characteristics of the rings, making it possible to more efficiently make a simple diagnosis. Further, by obtaining, in combination, images formed by the emission of ring light having such other forms from the display screen 5 of the mobile terminal 10A, and by obtaining a plurality of images having different ring light forms and performing image processing, it is possible to utilize the images as various diagnostic materials.

### Descriptions of Reference Numerals

- 1: Light source
- 1a: Light source separately provided
- 1b: Attaching part
- 1c: Liquid crystal panel
- 2: Camera
- 2a: Main camera
- 2b: Sub-camera
- 3: Transceiver function
- 5: Display screen
- 10: Simple diagnosis assisting device
- 10A: Mobile terminal
- 21a: Duplex ring light
- 21b: Simplex ring light
- a, b, c, d: Predetermined position

## Claims

1. A simple diagnosis assisting device for keratoconus and/or astigmatism, comprising:
a mobile terminal including a camera and a transceiver function, and integrally or separately provided with a light source for simultaneously projecting ring light onto both eyes;
the light source emitting the ring light upon being attached to the mobile terminal in a case of being separately provided to the mobile terminal, and emitting the ring light from the mobile terminal in a case of being integrally provided to the mobile terminal; and
the camera simultaneously capturing a projected image of both eyes onto which the ring light is projected.

2. The simple diagnosis assisting device according to claim 1, wherein
the light source is disposed toward a side permitting visual confirmation of a display of the mobile terminal.

3. The simple diagnosis assisting device according to claim 1 or 2, wherein
the ring light is multiplex ring light or simplex ring light.

4. The simple diagnosis assisting device according to any one of claims 1 to 3, wherein
the ring light is configured in a ring shape by a plurality of point light sources or is configured in a ring shape by a single linear light source.

5. The simple diagnosis assisting device according to any one of claims 1 to 4, wherein
the ring light has a color at a predetermined position on a contour thereof that is different from the color at other positions.

6. The simple diagnosis assisting device according to any one of claims 1 to 5, wherein
diagnosis assistance for the keratoconus, the astigmatism, and other ocular conditions is provided from a topography of an eyeball obtained on the basis of photographic data captured by the camera.

7. The simple diagnosis assisting device according to any one of claims 1 to 6, wherein
the mobile terminal incorporates an application for adjusting a contrast and/or an illuminance of the ring light in a case in which the light source is integrally provided to the mobile terminal, and
the light source incorporates a device for adjusting the contrast and/or the illuminance of the ring light in a case in which the light source is separately provided to the mobile terminal.

8. A diagnosis assisting system comprising:
the simple diagnosis assisting device described in any one of claims 1 to 7; and
a terminal that receives diagnosis assistance information transmitted from the simple diagnosis assisting device.
